# EUROPEAN PATENT SPECIFICATION

(11) **EP 4 205 140 B1**
(45) Date of publication and mention of the grant of the patent: **28.08.2024**
(21) Application number: 20820830.6
(22) Date of filing: 04.12.2020
(51) Int. Cl.: G16H 50/20, G08B 25/01, A61B 5/00, A61B 5/024, A61B 5/08, G16H 30/40, B60R 21/013, B60R 16/023

(54) **METHOD FOR A VEHICLE FOR DETERMINING AN INJURY INFORMATION AND VEHICLE ELECTRONIC CONTROL DEVICE FOR DETERMINING AN INJURY INFORMATION**
VERFAHREN FÜR EIN FAHRZEUG ZUR BESTIMMUNG EINER VERLETZUNGSINFORMATION UND EINES ELEKTRONISCHEN STEUERGERÄTS FÜR DAS FAHRZEUG ZUR BESTIMMUNG EINER VERLETZUNGSINFORMATION
PROCÉDÉ POUR VÉHICULE POUR LA DÉTERMINATION D'INFORMATIONS DE BLESSURES ET DISPOSITIF DE COMMANDE ÉLECTRONIQUE DE VÉHICULE POUR LA DÉTERMINATION D'INFORMATIONS DE BLESSURES

(30) Priority: 31.08.2020 IN 202041037440
(43) Date of publication of application: 05.07.2023
(73) Proprietor: Continental Automotive Technologies GmbH, 30175 Hannover (DE)
(72) Inventor: DAMODHARAN, Suchitra, 90411 Nürnberg (DE)
(74) Representative: Continental Corporation
(86) International application number: PCT/EP2020/084621
(87) International publication number: WO 2022/042868

(56) References cited:
- WO-A1-2020/136658
- US-A- 6 166 656
- US-A1- 2014 300 739

## Description

### Field of invention

The invention relates to a method for a vehicle for determining an injury information about an injury to a vehicle occupant after an accident. The invention further relates to a vehicle electronic control device for determining an injury information about an injury to a vehicle occupant after an accident.

### Description of the Related Art

The safety of occupants in vehicles assumes an important role in the development of vehicles. Safety approaches frequently include several measures for increasing driving safety, from a structural protection of the vehicle cabin, to occupant restraint systems like airbags and belt tightening systems, to an automated transmission of emergency calls with the aim of being able to better protect occupants in the vehicle in case of an accident.

Automatic emergency call methods and systems are well known, wherein in the event of a vehicular accident an emergency call is transmitted particularly to a rescue control center or the like provided with information related to the accident. Such systems are also known as eCall systems. This is intended to ensure that a rescue chain is set up as quickly as possible. To this end, usually information on the position and direction of travel of the vehicle involved in the accident is provided with the emergency call.

For the survival and the maximum achievable rehabilitation success of an injured occupant, the length of time from the accident to the first effective assistance and thus to the arrival of the professional rescue service and the quality of the initial treatment at the accident location as well as the subsequent clinical care are of decisive importance in emergency care. With the known emergency call methods and systems, a noticeable time saving can be achieved in the rescue phase after an accident of the vehicle by shortened search times or a shortened approach time. Such a system is known from WO 2020/136658 A1.

However, with the known emergency call methods and systems it can be regarded as disadvantageous that a disposition decision for rescue services to be deployed is left to the rescue control center and that no detailed information regarding crash and injury severity is provided.

### Summary of the Invention

In view of the foregoing, it is an object of the present invention to provide a method and a vehicle electronic control device for determining an injury information of a vehicle occupant after an accident of the vehicle, which ensure an accurate and proper determination of the injury information and thus enable a quick and efficient post-crash care and thus an enhanced safety of a vehicle occupant.

According to one aspect of the invention, a method for determining an injury information of a vehicle occupant after an accident of the vehicle is provided. The method comprising:
- sensing, by a vital sensor, at least one physiological signal of the occupant and providing a first output signal indicative of an internal trauma of the occupant to the vehicle electronic control device;
- providing to the vehicle electronic control device at least one second output signal from a vehicle internal sensor indicative of an external physical injury of the occupant; and
- determining, by the vehicle electronic control device, an emergency severity index indicative of a life-criticality status based on the first output signal and the second output signal.

The invention is based on the consideration that a quick and efficient post-crash care of an injured occupant can be achieved if an injury information of the occupant is determined, and wherein therefore an emergency severity index indicative of a life-criticality status is determined. The invention is further based on the consideration that and accurate and confident determination can be achieved if the determination is based also on post-crash data, and wherein the determination is based on a combination of physiological data or internal trauma data of the occupant and external physical injury data of the occupant.

In accordance with the present invention an accurate and proper determination of an injury information is enabled and thus a quick and efficient post-crash care and thus an enhanced safety of a vehicle occupant are ensured.

The steps of the method are preferably done in a post-crash phase, particularly immediately after the occurring of the crash or accident.

The external physical injury can be particularly an external bleeding of the occupant and/or a penetrateable injury of the occupant.

The method can use more than one vital sensor and preferably more than one physiological signal is sensed, particularly various physiological signals. Preferably, the one or more vital sensors are part of an occupant monitoring system that is capable of acquiring, particularly various, physiological signals. Furthermore, the method can use more than one vehicle internal sensor.

The vehicle internal sensor is an interior sensor situated in the vehicle, wherein the vehicle internal sensor particularly is integrated in the roof lining between the vehicle front seats and/or in an area close to the rear-view mirror of the vehicle.

Preferably, in a pre-crash phase a dormant stage of the vital sensor and the vehicle internal sensor is activated to prevent damage of the sensors due to the following crash or accident. After the crash or accident, i.e. in a post-crash phase, the respective sensors are re-activated. Thereby, a power reserve can be utilized to re-activate the sensors.

In case of an accident of the vehicle with more than one occupant within the vehicle, for each of the occupants an emergency severity index is determined.

Furthermore, in case of an accident of the vehicle with more than one occupant within the vehicle, it is preferably prioritized based on the age of the occupants. Accordingly, particularly children and elderly are prioritized when performing the method. Regarding the step of sensing, by a vital sensor, at least one physiological signal of the occupant and providing a first output signal indicative of an internal trauma of the occupant to the vehicle electronic control device, alternatively or additionally to the age of the occupants it is prioritized based on medical ailments in history of the occupants which can be retrieved from a storage unit of the vehicle electronic control device. Accordingly, particularly children and elderly and/or occupants with medical ailments in history are prioritized when performing this step. Regarding the step of providing to the vehicle electronic control device at least one second output signal from a vehicle internal sensor indicative of an external physical injury of the occupant, alternatively or additionally to the age of the occupants it is prioritized based on a status of a restraint means for the occupants. Accordingly, particularly children and elderly and/or occupants whose corresponding restraint means are ineffective or disabled are prioritized when performing this step.

According to an advantageous specific embodiment, the at least one physiological signal is at least one of respiration rate, heartbeat, heart rate, pulse rate, body temperature, oxygen saturation or brain wave.

According to another specific embodiment, the vehicle internal sensor is designed as an optical sensor, preferably as an imaging sensor, for example an interior camera. Particularly, the vehicle internal sensor is designed as a thermal imaging sensor, wherein by using a thermal imaging sensor also an internal injury of the occupant can be additionally be detected.

In case of an external bleeding of the occupant as an external physical injury particularly a video processing of blood detection on contact surfaces and the occupant can be used to derive the at least one second output signal.

Preferably in case of a penetrateable injury of the occupant the at least one second output signal is derived based on occupant state information in the pre-crash phase.

According to another specific embodiment, the method further comprising:
- sensing, by a crash sensor, at least one crash-related data and providing a third output signal indicative of a crash dynamic to the vehicle electronic control device;
- determining at least one of an impact location or a damage extend based on the third output signal and generating, by the vehicle electronic control device, a fourth output signal indicative of the impact location and/or the damage extend; and
- determining, by the vehicle electronic control device, the emergency severity index indicative of a life-criticality status based on the first output signal, the second output signal and the fourth output signal.

In this way, the determination of the emergency severity index further takes into account the impact location and/or a damage extend derived from a signal of a crash sensor. The determination of an injury information will be further precisionized in this was.

The step of sensing, by a crash sensor, at least one crash-related data and providing a third output signal indicative of a crash dynamic to the vehicle electronic control device is preferably done in the pre-crash phase and/or at the point when the crash or accident occurs, i.e. whereas the crash-phase.

In the step of determining at least one of an impact location or a damage extend only the impact location can be determined. Alternatively, only the damage extend can be determined. It is also alternatively possible that in this step an impact location and a damage extend are determined. In case of determining an impact location, the information of the one or more impact locations can also be used for the vehicle internal sensor to specify the regions of scanning. Furthermore, the determined one or more impact locations can be categorized based on the level of possibility of being an impact location. Different categories like 'High (Certain)', Medium (Probable)' and 'Low (Improbable)' can be used for this and can be assigned to the respective impact location.

Preferably, more than one crash sensor is used, and more than one crash-related data is sensed, particularly various crash-related data. The crash sensor can particularly be designed as an acceleration sensor, an angular rate sensor, a pressure sensor or a surrounding sensor like a radar sensor, a lidar sensor or an exterior camera.

In a preferred further embodiment, the at least one crash-related data is at least one of a vehicle acceleration, a collision velocity, a collision angle, or a pressure. Combinations of these data are also possible.

In a preferred further embodiment, the determining of at least one of the impact location or the damage extend is further based on at least one of the following information: a seat position of the occupant, a status of an occupant restraint system, presence of a mobile object within the vehicle cabin, or a design of the vehicle cabin. This information can preferably be determined already in a phase before the crash or accident.

According to another specific embodiment, the method further comprising:
- generating, by the vehicle electronic control device, a control signal for triggering a transmitting of an emergency call in response to the determined emergency severity index.

In a preferred further embodiment, the emergency call comprises at least one of the following emergency calls: emergency call for immediate assistance from nearby vehicle, emergency call for automatic collision notification system, emergency call for emergency medical service personnel, or emergency call to a personal contact notification of the occupant. So, in dependence of the determined emergency severity index an emergency call to a specific recipient is transmitted.

In a preferred further embodiment, the determined emergency severity index is provided with the emergency call. Thus, with the emergency call also the emergency severity index is transmitted and provided to the recipient.

In a preferred further embodiment, at least on of an occupant seat position or an occupant age is provided with the emergency call. Thus, with the emergency call also the occupant seat position and/or the occupant age is transmitted and provided to the recipient. Furthermore, also the number of occupants in the accident vehicle and the number of children or elderly, which can be derived particularly from the age of the occupant, can be provided with the emergency call.

According to another specific embodiment, the emergency severity index is stored in a storage unit of the vehicle electronic control device. In this way, necessary information is available for emergency response personnel at the location of the accident.

According to another aspect of the invention, a vehicle electronic control device for determining an injury information about an injury to a vehicle occupant after an accident is provided, wherein the vehicle electronic control device is configured to:
- obtain a first output signal indicative of an internal trauma of the occupant from a vital sensor sensing at least one physiological signal of the occupant;
- obtain at least one second output signal from a vehicle internal sensor indicative of an external physical injury of the occupant; and
- determine an emergency severity index indicative of a life-criticality status based on the first output signal and the second output signal.

The advantages and the specific and preferred embodiments described for the method according to the invention also apply accordingly to the vehicle electronic control device according to the invention.

### Brief Description of the Drawings

The drawings described herein are used to provide a further understanding for the present invention, and constitute a part of the present invention. The exemplary embodiments and descriptions of the present invention are used to explain the present invention, and do not constitute an improper limitation to the present invention. In the drawings:
FIG. 1 shows a flow chart of a method for determining an injury information of a vehicle occupant after an accident of the vehicle according to a preferred embodiment of the invention; and
FIG. 2 shows a flow chart of a method for determining the emergency severity index according to FIG. 1.

### Detailed Description

The following describes the present invention in detail with reference to the accompanying drawings and in combination with embodiments. It should be noted that, without conflicts, the embodiments in the present invention and features in the embodiments may be combined with each other. Parts corresponding to each other are always provided with the same reference signs in all figures.

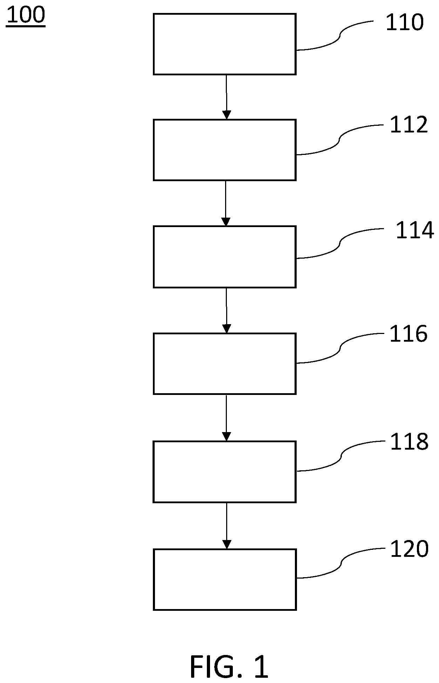

FIG. 1 shows a flow chart of a method 100 for determining an injury information of a vehicle occupant after an accident of the vehicle.

In step 110 crash-related data is sensed by a crash sensor whereas the crash or accident of the vehicle and a third output signal indicative of a crash dynamic is provided to a vehicle electronic control device which is designed as an airbag control device.

In step 112 an impact location and a damage extend are determined based on the third output signal and on a seat position of the occupant, wherein the seat position of the occupant was determined already in a phase before the crash or accident. A fourth output signal indicative of the impact location and the damage extend is then generated by the vehicle electronic control device.

In step 114, after occurring of the accident, several physiological signals of the occupant are sensed by different vital sensors, wherein these vital sensors are part of an occupant monitoring system, and a first output signal indicative of an internal trauma of the occupant is provided to the vehicle electronic control device.

In step 116 a second output signal from a vehicle internal sensor, designed as an interior camera, indicative of an external physical injury of the occupant like a bleeding is provided to the vehicle electronic control device.

In step 118 an emergency severity index indicative of a life-criticality status is determined by the vehicle electronic control device based on the first output signal, the second output signal and the fourth output signal.

In this way, an accurate and confident determination of a life-criticality status of the occupant can be achieved.

In case of an accident of the vehicle with more than one occupant within the vehicle, for each of the occupants an emergency severity index is determined by the described steps.

In step 120 a control signal for triggering a transmitting of an emergency call in response to the determined emergency severity index is generated by the vehicle electronic control device and the emergency call is transmitted to a respective recipient. With the emergency call the position of the vehicle, the number of occupants of the vehicle, the number of children or elderly among the occupants, the seat position of the occupant and the determined emergency severity index for the occupant is provided or transmitted. In this step the provided or transmitted information is also stored in a storage unit of the vehicle electronic control device.

In this way, a quick and efficient post-crash care of an injured occupant can be achieved based on the accurate and confident determination of the emergency severity index indicative of the life-criticality status of the occupant.

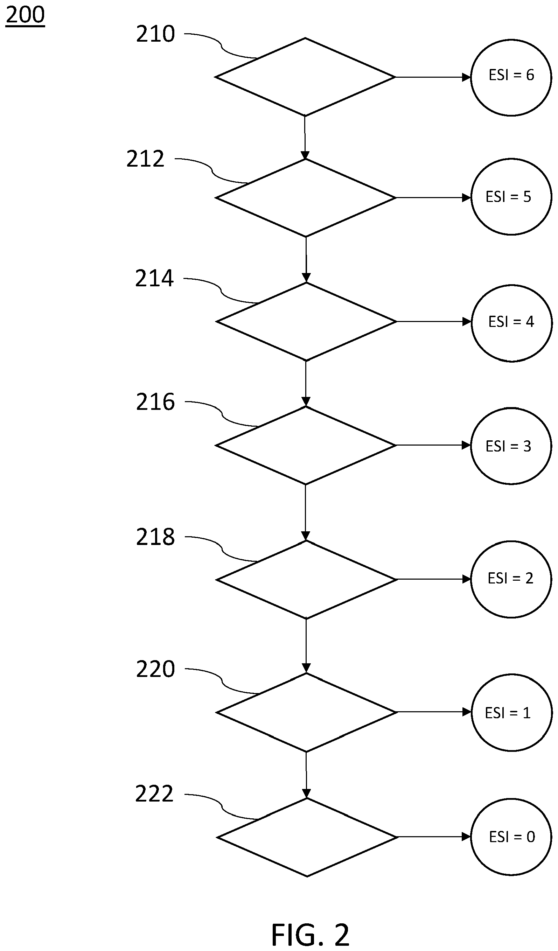

FIG. 2 shows a flow chart of a method 200 for determining the emergency severity index according to FIG. 1.

In step 210 it is determined if the occupant is alive. This determination is done based on the sensed physiological signals or the first output signal. If none vital can be sensed or detected, then the value 6 is assigned as the emergency severity index (ESI).

If at least one of the vitals is being sensed or detected, then in step 212 it is determined if the occupant is dying. This determination is done based on the sensed physiological signals respiration rate and heart rate or the respective first output signal. If these vitals fading, i.e. reach critical levels, then the value 5 is assigned as the emergency severity index (ESI).

If these vitals do not fade, i.e. do not reach critical levels, then in step 214 the criticality of the occupant vitals is determined. This determination is done based on the sensed physiological signals respiration rate, heart rate and oxygen saturation or the respective first output signal. If these vitals are below an acceptable predetermined vital threshold, then the value 4 is assigned as the emergency severity index (ESI).

If these vitals are not below the predetermined vital threshold, then in step 216 the neurological state of consciousness of the occupant is determined according to the known Glasgow Coma Scale based on the sensed physiological signals or the first output signal. If the determined Glasgow Coma Scale is below a predetermined limit value, then the value 3 is assigned as the emergency severity index (ESI).

If the determined Glasgow Coma Scale is above the predetermined limit value, then in step 218 an occupant injury score is determined based on the number of physical injuries and their severity provided by the second output signal. If the determined occupant injury score is above a predetermined injury threshold, then the value 2 is assigned as the emergency severity index (ESI).

If the determined occupant injury score is below the predetermined injury threshold, then in step 220 an occupant injury is estimated based on a probable impact analysis and an injury estimate which is based on the fourth output signal. If an injury to the occupant is assumed, then the value 1 is assigned as the emergency severity index (ESI).

If no injury to the occupant is assumed, then in step 222 it is ascertained that no medical assistance is needed and the value 0 is assigned as the emergency severity index (ESI).

In the event that all of the information, data or signals are not accessible, then the value 9 is assigned as the emergency severity index (ESI).

## Claims

1. A method (100) for a vehicle for determining an injury information of a vehicle occupant after an accident of the vehicle, the method (100) comprising the steps of:
- sensing (114), by a vital sensor, at least one physiological signal of the occupant and providing a first output signal indicative of an internal trauma of the occupant to the vehicle electronic control device;
- providing (116) to the vehicle electronic control device at least one second output signal from a vehicle internal sensor indicative of an external physical injury of the occupant; **characterized in that** the method further comprises the step of:
- determining (118, 200), by the vehicle electronic control device, an emergency severity index indicative of a life-criticality status based on the first output signal and the second output signal.

2. A method (100) according to claim 1, wherein the at least one physiological signal is at least one of respiration rate, heartbeat, heart rate, pulse rate, body temperature, oxygen saturation or brain wave.

3. A method (100) according to claim 1 or 2, wherein the vehicle internal sensor is designed as an optical sensor, preferably as an imaging sensor.

4. A method (100) according to one of preceding claims, further comprising:
- sensing (110), by a crash sensor, at least one crash-related data and providing a third output signal indicative of a crash dynamic to the vehicle electronic control device;
- determining (112) at least one of an impact location or a damage extend based on the third output signal and generating, by the vehicle electronic control device, a fourth output signal indicative of the impact location and/or the damage extend; and
- determining (118, 200), by the vehicle electronic control device, the emergency severity index indicative of a life-criticality status based on the first output signal, the second output signal and the fourth output signal.

5. A method (100) according to claim 4, wherein the at least one crash-related data is at least one of a vehicle acceleration, a collision velocity, a collision angle, or a pressure.

6. A method (100) according to claim 4 or 5, wherein the determining (112) of at least one of the impact location or the damage extend is further based on at least one of the following information: a seat position of the occupant, a status of an occupant restraint system, presence of a mobile object within the vehicle cabin, or a design of the vehicle cabin.

7. A method (100) according to one of preceding claims, further comprising:
- generating (120), by the vehicle electronic control device, a control signal for triggering a transmitting of an emergency call in response to the determined emergency severity index.

8. A method (100) according to claim 7, wherein the emergency call comprises at least one of the following emergency calls: emergency call for immediate assistance from nearby vehicle, emergency call for automatic collision notification system, emergency call for emergency medical service personnel, or emergency call to a personal contact notification of the occupant.

9. A method (100) according to claim 7 or 8, wherein the determined emergency severity index is provided with the emergency call.

10. A method (100) according to one of claims 7 to 9, wherein at least on of an occupant seat position or an occupant age is provided with the emergency call.

11. A method (100) according to one of preceding claims, wherein the emergency severity index is stored in a storage unit of the vehicle electronic control device.

12. A vehicle electronic control device for determining an injury information about an injury to a vehicle occupant after an accident, wherein the vehicle electronic control device is configured to:
- obtain a first output signal indicative of an internal trauma of the occupant from a vital sensor sensing at least one physiological signal of the occupant;
- obtain at least one second output signal from a vehicle internal sensor indicative of an external physical injury of the occupant; **characterized in that** the vehicle electronic control device is further configured to:
- determine an emergency severity index indicative of a life-criticality status based on the first output signal and the second output signal.

## Patentansprüche

1. Verfahren (100) für ein Fahrzeug zum Bestimmen einer Verletzungsinformation eines Fahrzeuginsassen nach einem Unfall des Fahrzeugs, wobei das Verfahren (100) die folgenden Schritte umfasst:
- Erfassen (114) mindestens eines physiologischen Signals des Insassen durch einen Vital-Sensor und Bereitstellen eines ersten Ausgangssignals, das kennzeichnend für ein internes Trauma des Insassen ist, an die elektronische Steuereinrichtung des Fahrzeugs;
- Bereitstellen (116) mindestens eines zweiten Ausgangssignals von einem fahrzeuginternen Sensor, das kennzeichnend für eine äußere körperliche Verletzung des Insassen ist, an die elektronische Steuereinrichtung des Fahrzeugs; **dadurch gekennzeichnet, dass** das Verfahren ferner den folgenden Schritt umfasst:
- Bestimmen (118, 200), durch die elektronische Steuereinrichtung des Fahrzeugs, eines Notfall-Schweregrad-Indexes, der basierend auf dem ersten Ausgangssignal und dem zweiten Ausgangssignal kennzeichnend für einen lebensbedrohenden Zustand ist.

2. Verfahren (100) nach Anspruch 1, wobei das mindestens eine physiologische Signal eine Atemfrequenz und.oder ein Herzschlag und/oder eine Herzfrequenz und/oder eine Pulsfrequenz und/oder eine Körpertemperatur und/oder eine Sauerstoffsättigung und/oder ein Gehirnstrom ist.

3. Verfahren (100) nach Anspruch 1 oder 2, wobei der fahrzeuginterne Sensor als optischer Sensor, bevorzugt als Bilderzeugungssensor, ausgebildet ist.

4. Verfahren (100) nach einem der vorhergehenden Ansprüche, das ferner umfasst:
- Erfassen (110) von mindestens einem Crash-bezogenen Datenwert durch einen Crashsensor und Bereitstellen eines dritten Ausgangssignals, das kennzeichnend für eine Crashdynamik ist, an die elektronische Steuereinrichtung des Fahrzeugs;
- Bestimmen (112) einer Aufprallposition und/oder eines Schadensausmaßes basierend auf dem dritten Ausgangssignal und Erzeugen eines vierten Ausgangssignals, das die Aufprallposition und/oder das Schadensausmaß angibt, durch die elektronische Steuereinrichtung des Fahrzeugs; und
- Bestimmen (118, 200), durch die elektronische Steuereinrichtung des Fahrzeugs, des Notfall-Schweregrad-Indexes, der basierend auf dem ersten Ausgangssignals, dem zweiten Ausgangssignal und dem vierten Ausgangssignal kennzeichnend für einen lebensbedrohenden Zustand ist.

5. Verfahren (100) nach Anspruch 4, wobei die mindestens einen crashbezogenen Daten eine Fahrzeugbeschleunigung und/oder eine Kollisionsgeschwindigkeit und/oder ein Kollisionswinkel und/oder ein Druck sind.

6. Verfahren (100) nach Anspruch 4 oder 5, wobei das Bestimmen (112) der Aufprallposition und/oder des Schadensausmaßes ferner auf mindestens einer der folgenden Informationen basiert: einer Sitzposition des Insassen, einem Status eines Insassen-Rückhaltesystems, dem Vorhandensein eines beweglichen Objekts in der Fahrzeugkabine oder einer Konstruktion der Fahrzeugkabine.

7. Verfahren (100) nach einem der vorhergehenden Ansprüche, das ferner umfasst:
- Erzeugen (120), durch die elektronische Steuereinrichtung des Fahrzeugs, eines Steuersignals zum Auslösen des Sendens eines Notrufs als Reaktion auf den bestimmten Notrufschweregrad-Index.

8. Verfahren (100) nach Anspruch 7, wobei der Notruf zumindest einen der folgenden Notrufe umfasst: Notruf zur sofortigen Hilfeleistung durch ein in der Nähe befindliches Fahrzeug, Notruf für ein automatisches Kollisionsbenachrichtigungssystem, Notruf für medizinisches Notfallpersonal oder Notruf für eine persönliche Kontaktbenachrichtigung des Insassen.

9. Verfahren (100) nach Anspruch 7 oder 8, wobei der bestimmte Notfall-Schweregrad-Index mit dem Notruf bereitgestellt wird.

10. Verfahren (100) nach einem der Ansprüche 7 bis 9, wobei mit dem Notruf eine Sitzposition des Insassen und/oder ein Alter des Insassen bereitgestellt wird.

11. Verfahren (100) nach einem der vorhergehenden Ansprüche, wobei der Notfall-Schweregrad-Index in einer Speichereinheit der elektronischen Steuereinrichtung des Fahrzeugs gespeichert wird.

12. Elektronische Steuereinrichtung eines Fahrzeugs zum Bestimmen einer Verletzungsinformation über eine Verletzung eines Fahrzeuginsassen nach einem Unfall, wobei die elektronische Steuereinrichtung des Fahrzeugs dazu ausgelegt ist:
- ein erstes Ausgangssignal, das für ein internes Trauma des Insassen kennzeichnend ist, von einem Vitalsensor zu erhalten, der mindestens ein physiologisches Signal des Insassen erfasst;
- mindestens ein zweites Ausgangssignal von einem fahrzeuginternen Sensor zu erhalten, das kennzeichnend für eine äußere körperliche Verletzung des Insassen ist; **dadurch gekennzeichnet, dass** die elektronische Steuereinrichtung des Fahrzeugs ferner dazu ausgelegt ist:
- einen Notfall-Schweregrad-Index zu bestimmen, der basierend auf dem ersten Ausgangssignal und dem zweiten Ausgangssignal kennzeichnend für einen lebensbedrohenden Zustand ist.

## Revendications

1. Procédé (100) pour un véhicule permettant de déterminer une information de blessure d'un occupant du véhicule après un accident du véhicule, le procédé (100) comprenant les étapes suivantes :
- détecter (114), par un capteur vital, au moins un signal physiologique de l'occupant et fournir un premier signal de sortie indicatif d'une blessure interne de l'occupant au dispositif de commande électronique du véhicule ;
- fournir (116) au dispositif de commande électronique du véhicule au moins un deuxième signal de sortie provenant d'un capteur interne du véhicule indicatif d'une blessure physique externe de l'occupant ; **caractérisé en ce que** le procédé comprend en outre l'étape suivante :
- déterminer (118, 200), par le dispositif de commande électronique du véhicule, un indice de gravité d'urgence indicatif d'un état vital critique sur la base du premier signal de sortie et du deuxième signal de sortie.

2. Procédé (100) selon la revendication 1, dans lequel l'au moins un signal physiologique est au moins l'un parmi la fréquence respiratoire, les battements cardiaques, la fréquence cardiaque, le pouls, la température corporelle, la saturation en oxygène ou les ondes cérébrales.

3. Procédé (100) selon la revendication 1 ou la revendication 2, dans lequel le capteur interne du véhicule est conçu sous la forme d'un capteur optique, de préférence sous a forme d'un capteur d'imagerie.

4. Procédé (100) selon l'une des revendications précédentes, comprenant en outre les étapes suivantes :
- détecter (110), par un capteur d'accident, au moins une donnée liée à l'accident et fournir un troisième signal de sortie indicatif d'une dynamique d'accident au dispositif de commande électronique du véhicule ;
- déterminer (112) au moins l'un des points d'impact ou l'étendue des dommages sur la base du troisième signal de sortie, et générer, par le dispositif de commande électronique du véhicule, un quatrième signal de sortie indicatif du point d'impact et/ou de l'étendue des dommages ; et
- déterminer (118, 200), par le dispositif de commande électronique du véhicule, l'indice de gravité d'urgence indicatif d'un état vital critique sur la base du premier signal de sortie, du deuxième signal de sortie et du quatrième signal de sortie.

5. Procédé (100) selon la revendication 4, dans lequel l'au moins une donnée liée à l'accident est au moins l'une des données suivantes : une accélération du véhicule, une vitesse de collision, un angle de collision ou une pression.

6. Procédé (100) selon la revendication 4 ou la revendication 5, dans lequel la détermination (112) d'au moins une des données parmi le point d'impact ou l'étendue des dommages est en outre basée sur au moins une des informations suivantes : la position du siège de l'occupant, l'état d'un système de retenue de l'occupant, la présence d'un objet mobile dans l'habitacle du véhicule ou la conception de l'habitacle du véhicule.

7. Procédé (100) selon l'une des revendications précédentes, comprenant en outre l'étape suivante :
- générer (120), par le dispositif de commande électronique du véhicule, un signal de commande pour déclencher la transmission d'un appel d'urgence en réponse à l'indice de gravité d'urgence déterminé.

8. Procédé (100) selon la revendication 7, dans lequel l'appel d'urgence comprend au moins un des appels d'urgence suivants : un appel d'urgence pour une assistance immédiate d'un véhicule proche, un appel d'urgence pour un système de notification automatique de collision, un appel d'urgence pour un personnel de service médical d'urgence, ou un appel d'urgence pour une notification à un contact personnel de l'occupant.

9. Procédé (100) selon la revendication 7 ou la revendication 8, dans lequel l'indice de gravité d'urgence déterminé est fourni avec l'appel d'urgence.

10. Procédé (100) selon l'une des revendications 7 à 9, dans lequel au moins l'un des éléments suivants, à savoir la position du siège de l'occupant ou l'âge de l'occupant, est fourni avec l'appel d'urgence.

11. Procédé (100) selon l'une des revendications précédentes, dans lequel l'indice de gravité d'urgence est stocké dans une unité de stockage du dispositif de commande électronique du véhicule.

12. Dispositif de commande électronique d'un véhicule pour déterminer une information de blessure subie par un occupant du véhicule après un accident, où le dispositif de commande électronique du véhicule est configuré pour :
- obtenir un premier signal de sortie indicatif d'une blessure interne de l'occupant à partir d'un capteur vital détectant au moins un signal physiologique de l'occupant ;
- obtenir au moins un deuxième signal de sortie d'un capteur interne du véhicule indicatif d'une blessure physique externe de l'occupant ; **caractérisé en ce que** le dispositif de commande électronique du véhicule est en outre configuré pour :
- déterminer un indice de gravité d'urgence indiquant un état vital critique sur la base du premier signal de sortie et du deuxième signal de sortie.
